# EUROPÄISCHE PATENTSCHRIFT

(11) **EP 3 840 600 B1**
(45) Veröffentlichungstag und Bekanntmachung des Hinweises auf die Patenterteilung: **27.07.2022**
(21) Anmeldenummer: 19748819.0
(22) Anmeldetag: 31.07.2019
(51) Int. Cl.: A41B 11/00, A61F 13/08, A41B 11/02, A41B 11/14, A41D 13/00, A63B 21/055

(54) **SPORTSOCKEN ODER SPORTBANDAGE**
ATHLETIC SOCK OR ATHLETIC BANDAGE
CHAUSSETTE DE SPORT OU BANDAGE DE SPORT

(30) Priorität: 22.08.2018 CH 10152018; 12.02.2019 CH 1672019
(43) Veröffentlichungstag der Anmeldung: 30.06.2021
(73) Patentinhaber: X-Technology Swiss GmbH, 8832 Wollerau (CH)
(72) Erfinder: LAMBERTZ, Bodo, 8853 Wilen bei Wollerau Schwyz (CH)
(74) Vertreter: Prins Intellectual Property AG
(86) Internationale Anmeldenummer: PCT/EP2019/070683
(87) Internationale Veröffentlichungsnummer: WO 2020/038694

(56) Entgegenhaltungen:
- EP-A2- 1 323 397
- WO-A1-2016/157227
- US-B1- 6 311 334
- US-B1- 9 387 125

## Beschreibung

### Technisches Gebiet

Die vorliegende Erfindung beschreibt ein Sportsocken, welches aus einem gewirkten oder gestrickten Basistextil gebildet ist, einen röhrenförmigen Schaft mit einem Bund aufweist, wobei im Verlauf des Schafts Kompressionsmittel angeordnet sind, welche eine vom Basistextil abweichende Struktur, Garndicke oder Garnmaterial aufweisen.

### Stand der Technik

Aus dem Stand der Technik bekannte Kompressionskleidung und entsprechend auch Sportsocken und Kompressionsstrümpfe erzeugen Druck auf die Haut und das darunterliegende Gewebe, insbesondere eines umschlossenen Beines. Kompressionsstrümpfe werden aus medizinischen, kosmetischen oder prophylaktischen Gründen eingesetzt, beispielsweise können Krampfadern (Varizen) oder Beinvenenthrombosen behandelt bzw. verhindert werden. Durch die prophylaktische Anwendung kann das Risiko einer Thrombose beispielsweise auf Langstreckenflügen wesentlich reduziert werden. Zudem findet Kompressionskleidung, insbesondere Kompressionsstrümpfe, im Sport Anwendung, beispielsweise beim Nordic-Walking oder Marathonlaufen.

Die bekannte Kompressionskleidung übt einen flächigen Druck auf den Körper, insbesondere im Bereich des Unterschenkels aus. Es wird eine gleichmässige Druckverteilung durch die Kompressionswirkung erreicht, welche aber zu einer Einschnürung führt, wodurch die Blutversorgung behindert wird. Dies ist vor allem im Sportbereich nicht gewünscht, sodass bereits länger bekannt ist, dass eine vollflächige Kompression für den Einsatz beim Sport ungeeignet ist.

Man hat versucht, durch die Anordnung mehrerer Strumpfbereiche von einer vollflächigen Kompression des Unterschenkels durch einen Strumpf wegzukommen.

Ein Sporttextil mit Kompressionswirkung, insbesondere ein Sportstrumpf ist aus der US2012102625 bekannt, wobei mindestens ein Kompressionsmittel in Form eines, den Sportstrumpf auf einer Höhe umlaufenden Bandes, welches aus einem elastischen Material derart hergestellt ist, dass eine lokale Kompressionswirkung resultiert.

In WO2018136867 wurde versucht, mehrere Kompressionsmittel lokal entlang eines gestrickten Sportstrumpfes verteilt anzuordnen, um eine gewünschte Kompressionswirkung zu erreichen, wobei der Sportstrumpf einfach herstellbar sein soll. Es werden unterschiedliche Strickverfahren eingesetzt und verschiedene Garne, wobei auch Bestandteile vernäht sein können. Wie dem Fachmann bekannt, werden Rippen oder Erhöhungen im Bereich der Kompressionsmittel angeordnet, welche eine Kompressionswirkung entfalten können.

Wie auch in der WO2010046130 beschrieben, muss bei einem optimierten Sporttextil durch geeignete Anordnung der Kompressionsmittel, dafür gesorgt werden, dass ein Teil der Kapillaren nicht durch Stege, Erhöhungen oder Rippen der Kompressionsmittel, die in Richtung der Hautoberfläche ragend gebildet sind, komprimiert werden. Die Blutzirkulation sollte möglichst ungestört bleiben, damit auch die Kühlung des Organismus, vor allem bei sportlicher Betätigung möglichst gut bleibt. Entsprechend hat sich durchgesetzt, Kompressionsmittel und Bereich ohne Kompression nebeneinander anzuordnen, was zu ausreichenden Kompressionswirkungen führt. Das resultierende Kompressionsergebnis ist aber teilweise unbefriedigend, weshalb an der Gestaltung der Kompressionsmittel Entwicklungsbedarf bestand. Es wäre aber auch vorteilhaft, wenn störende Muskelvibrationen beseitigt werden könnten, um die Stabilität des Unterschenkels eines Kompressionsstrumpfträgers oder im Bereich eines Ellenbogen- oder Kniegelenks zu erhöhen.

Dies hat man in der EP1323397 versucht, indem spiralförmig umlaufende Kompressionsmittel in Form von zwei gegenläufige Helices verwendet wurden, welche von einem Fussrückenbereich in Richtung eines Strumpfbundes den Strumpfschaft umlaufend ausgestaltet sind. Die damit erreichbare Kompression konnte aber die Muskelvibrationen im Bereich der Waden-, Schienbeinmuskeln und der Sprunggelenke nicht ausreichend verringern oder beseitigen. Die resultierende Stützwirkung ist bei einer solchen Lösung nahezu nicht vorhanden.

### Darstellung der Erfindung

Die vorliegende Erfindung hat sich zur Aufgabe gestellt einen Sportsocken oder Sportbandage als Kompressionsstrumpf derart zu verbessern, dass die Kompressionswirkung erhöht und zusätzlich mindestens im Bereich eines Schaftes und eines Gelenks zusätzlich eine stabilisierende Stützwirkung erreicht wird.

Es sollen beispielsweise die Beinmuskeln fixiert und Muskelvibrationen weitgehend verhindert werden, wodurch die sportliche Leistungsfähigkeit dauerhaft verbessert ist.

Diese Aufgabe wird durch einen Sportsocken oder Sportbandage mit einem röhrenförmigen Schaft gelöst, wobei spezifisch ausgestaltete lokal entlang des Schaftes verteilte mehrere Kompressionsmittel eingesetzt werden.

Weitere vorteilhafte Gestaltungen des Sportsockens, insbesondere am Beispiel eines Kompressionsstrumpfes mit Kompressionsmitteln erläutert, sind beschrieben und in den abhängigen Ansprüchen beansprucht.

Als Sportsocken bzw. Kompressionsstrumpf oder Sportbandage wird hier ein etwa bis zum Knie eines Benutzers ausgeführter Strumpf oder Socken verstanden, welcher üblicherweise ein Basisgestrick oder - gewirke umfasst und bevorzugt für den Einsatz bei sportlicher Betätigung und nicht für medizinische Anwendungen benutzt wird. Neben den Kompressionsmitteln sind zusätzliche Stützmittel in Form von Polstern vorteilhaft.

### Kurze Beschreibung der Zeichnungen

Ein bevorzugtes Ausführungsbeispiel des Erfindungsgegenstandes wird nachstehend im Zusammenhang mit den anliegenden Zeichnungen beschrieben.
- Figur 1: zeigt eine perspektivische schematische Ansicht eines Sportsockens, am Beispiel eines Kompressionsstrumpfes mit Kompressionsmitteln von vorne, während
- Figur 1b eine 3d: Darstellung gemäss Figur 1a mit Schwerpunkt Kompressionsmittel auf Vorder- und Rückseite zeigt und in den
- Figuren 1c bis 1g: sind schematische Ansichten verschiedener Kompressionsstrümpfe mit unterschiedliche vielen bzw. unterschiedlich angeordneten Kompressionsmitteln gezeigt.
- Figur 2a: zeigt eine Ansicht eines Kompressionsstrumpfes mit Kompressionsmitteln und Zusatzpolstern im angezogenen Zustand, über einen Unterschenkel übergestreift, während
- Figur 2b: einen Kompressionsstrumpf gemäss Figur 2a im plan ausgelegten nicht getragenen Zustand nach Entnahme aus der Strick- oder Wirkmaschine zeigt.

### Beschreibung

Am Beispiel eines Sportsockens 1 bzw. Kompressionsstrumpfes 1 aus einem Basistextil, bevorzugt für den Einsatz eines Benutzers bei sportlicher Betätigung, bei welcher man längere Strümpfe oder Kniestrümpfe trägt, wird im Folgenden beispielhaft als Sportsocken 1 beschrieben.

Der Kompressionsstrumpf 1 bzw. das Basistextil ist aus einem Gestrick oder Gewirke hergestellt und weist hier eine tragende Hülle aus einem Grundgestrick 10 auf. Von einem Strumpfbund 11 verläuft der Kompressionsstrumpf 1 in einen Strumpfschaft 12, welcher einen Wadenbereich 120 und einen Sprunggelenksbereich 121 bildet. Der Wadenbereich 120 und der Sprunggelenksbereich 121 befinden sich jeweils auf Höhe der entsprechenden Fussbereiche bei angezogenem Kompressionsstrumpf 1 eines Benutzers. Auf der vom Strumpfbund 11 abgewandten Seite des Strumpfschaftes 12 schliessen sich ein Fussrückenbereich 14, ein Zehenbereich 16, eine Strumpfsohle 15 und ein Fersenbereich 13 an, wobei alle Kompressionsstrumpfteile einen, einen Unterschenkel umschliessenden, zur Strumpfsohle 15 geschlossenen Kompressionsstrumpf 1 bilden. Möglich wären aber auch Kompressionsstrümpfe 1, die auf der vom Strumpfbund 11 gegenüberliegenden Seite offen sind.

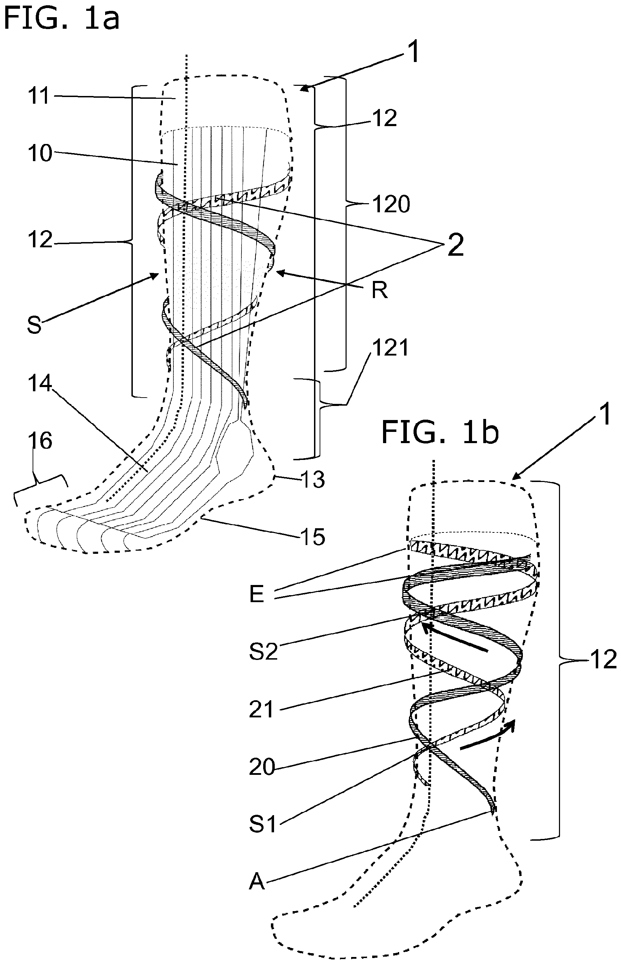

Bevorzugt ist der Kompressionsstrumpf 1 aus einem Basisgestrick auf einer Strickmaschine möglichst einstückig hergestellt. Es können aber auch mehrere Strickteile hergestellt und anschliessend zu einem Kompressionsstrumpf 1 verbunden werden. Die schematische Ansicht gemäss Figur 1a ist aus Richtung einer Textilvorderseite S dargestellt, während eine Textilrückseite R teilweise verdeckt ist. Hier ist eine zentrale Verlaufslinie des Kompressionsstrumpfes 1 gestrichelt angedeutet, welche die Textilvorderseite S vom Fussrückenbereich 14 bis zum Strumpfbund 11 mittig durchläuft. Üblicherweise ist der Strumpfbund 11 in einem vom Grundgestrick 10 abweichenden Strickmuster und/oder Garn hergestellt.

Die hier beschriebenen Kompressionsstrümpfe sind vorteilhaft einsetzbar im Bereich Wintersport, dem Jagdsport oder sonstigen Aktivitäten, bei welchen längere oder Kniestrümpfe vorteilhaft sind. Dauerhaft wird eine Kompressionswirkung und eine muskelstützende Wirkung erzielt. Durch die muskelstützende Wirkung wird die Stabilität des Unterschenkels im Kompressionsstrumpf 1 verbessert und damit die Leistungsfähigkeit des Fusses. Trotz Einsatz derartiger Kompressionsmittel 2 wird die Blutzirkulation nicht nachteilig verändert, da noch ausreichend viele Bereiche des Unterschenkels wenig bis gar nicht komprimiert werden.

Entlang des Kompressionsstrumpfes 1 sind Kompressionsmittel 2 angeordnet. Diese Kompressionsmittel 2 sind hier in Form mehrerer gegenläufiger Helices 20, 21 oder Wendeln gestaltet, welche den Strumpfschaft 12 linksgängig oder rechtsgängig umgeben. Die Helices 20, 21 sind mindestens vom Sprunggelenksbereich 120 den Strumpfschaft 12 umlaufend in Richtung Strumpfbund 11 gewunden und kreuzen sich mindestens einmal.

Bevorzugt werden die Kompressionsmittel 2 von mindestens zwei sich mehrfach kreuzenden Helices 20, 21 mit unterschiedlichen Windungsrichtungen gebildet, wie hier dargestellt.

Es konnte hier neben einer gesteigerten Kompressionswirkung eine Muskelunterstützende bzw. -haltende Wirkung erzielt werden. Die Helices 20, 21 sind bevorzugt mit vom Grundgestrick 10 abweichendem Gestrick oder Gewirke ausgeführt oder weisen ein anderes Material auf, als das Garn des Grundgestrickes 10.

In Figur 1b ist ein Kompressionsstrumpf 1 gezeigt, welcher entlang seines äusseren Umfangs entlang des Strumpfschaftes 12 eine erste Helix 20, welche hier linksgängig ist und eine zweite Helix 21, welche hier rechtsgängig ausgestaltet ist, aufweist. Mit Pfeilen sind die unterschiedlichen Umlaufrichtungen der Windungen markiert.

Auch, wenn der Strumpfschaft 12 nicht zylindrisch ist, sondern eher konisch, wird hier der Begriff Helix verwendet, da sich die Kompressionsmittel 2 wendelartig als Kurve um den angenähert zylindrischen Strumpfschaft 12 winden. Jede Helix 20, 21 startet jeweils von einem Startpunkt A und umschliesst den Strumpfschaft 12 hier etwa zweimal bis zum einem Endpunkt E. Entlang der Helices 20, 21 schneiden sich die Windungen an einem ersten Schnittpunkt S1 und einem zweiten Schnittpunkt S2 damit zweimal. Die Schnittpunkte S1, S2 sind hier auf der Textilvorderseite S angeordnet und liegen entlang der zentralen Verlaufslinie des Kompressionsstrumpfes 1. Durch die unterschiedliche Windungsrichtungen der Helices 20, 21 schneiden sich die beiden Helices entsprechend auch an zwei Schnittpunkten auf der Textilrückseite R.

Die Helices 20, 21 sind zur Erreichung einer Kompression, wie im Bereich der Kompressionsmittel an den Gestricken dem Fachmann bekannt, durch verschiedene Garne, Garndicken, angeformte oder befestigte Erhebungen und/oder geeignete Strickmuster definiert und weisen einen bandartigen Charakter auf. Die Helices 20, 21 sind dabei in das Grundgestrick 10, beispielsweise durch eine Strickmaschine direkt bei der Herstellung des Kompressionsstrumpfes 1 mit eingearbeitet.

In Abwandlungen können mehr als zwei Helices 20, 21 verwendet werden, von welchen nur einige gegenläufig sind und in Längsrichtung des Strumpfschaftes 12 versetzt angeordnet sein können. So könnte eine linksgängige und n (n>1) rechtsgängige Helices oder umgekehrt in Längsrichtung versetzt angeordnet werden.

Generell können die verschiedenen Helices 20, 21 jeweils vom selben Startpunkt A ausgehen und/oder am selben Endpunkt E enden.

Vorteilhaft ist es, wenn beide Helices 20, 21 bzw. alle Helices mehr als 1.5 volle Umdrehungen, insbesondere mehr als zwei volle Umläufe um den Strumpfschaft 12 aufweisen. Die Muskelfixierende Wirkung wird dadurch erhöht.

Durch die Helices 20, 21 wird der Blutfluss durch die Venen zurück zum Herzen unterstützt und die Unterschenkelmuskeln werden fixiert, wodurch die Stabilität des Unterschenkels erhöht wird.

Die Steigungen der Helices 20, 21 können identisch oder geringfügig abweichend gestaltet werden. Die Ganghöhe, als Höhe einer vollen Umdrehung einer Helix um den Strumpfschaft 12 kann entsprechend gleich oder geringfügig abweichend ausgestaltet sein, wobei mehrere Schnittpunkte der Helices 20, 21 auftreten sollen. Wenn die Ganghöhe mindestens einem Viertel der Länge zwischen dem Rand des Strumpfbundes 11 und dem unteren Rand der Strumpfsohle 15 entspricht, kann sich eine optimale Stützwirkung bei optimaler Kompressionswirkung gleichzeitig entfalten.

Eine sehr gute Stützwirkung wird erreicht, wenn die mindestens zwei Helices 20, 21 mehr als zwei Kreuzungspunkte S1 entlang ihrer Verläufe aufweisen, wobei die Kreuzungspunkte S1 auf der Textilvorderseite S und/oder der Textilrückseite R angeordnet sein können.

Da der Krümmungsradius des Strumpfschaftes 12 aufgrund der leichten Konizität des Strumpfschaftes 12 nicht konstant ist, ist auch die Krümmung der Helices 20, 21 nicht konstant. Bei Herstellung eines Kompressionsstrumpfes auf einer Strickmaschine können die gewählten Strickmuster des Grundgestrickes 10 und des Gestrickes im Bereich der Helices 20, 21 aber entsprechend abgestimmt werden.

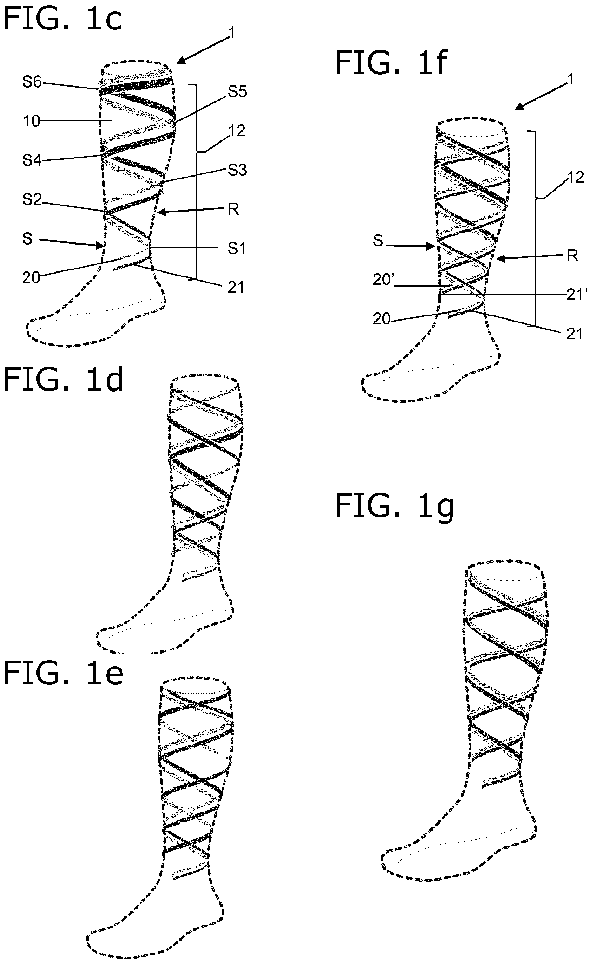

Wie in den Kompressionsstrümpfen 1 gemäss der Figuren 1c bis 1g gezeigt, können zwei Helices 20, 21 auf der Textilvorderseite S und der Textilrückseite R kreuzend angeordnet sein oder es werden drei Helices 20, 20', 21, 21', den Schaft 12 umlaufend gewählt, wie in den Figuren 1d und 1e, wobei sich die Helices an der Innenseite oder der Aussenseite des Schafts 12 kreuzen. Wahlweise können auch jeweils vier Helices, von denen sich jeweils zwei Paare überkreuzen gewählt werden, wie in Figuren 1f und 1g gezeigt, wobei Kreuzungspunkte auf der Textilvorderseite S und/oder Textilrückseite R und/oder der Schaftinnenseite und/oder der Schaftaussenseite angeordnet sein können.

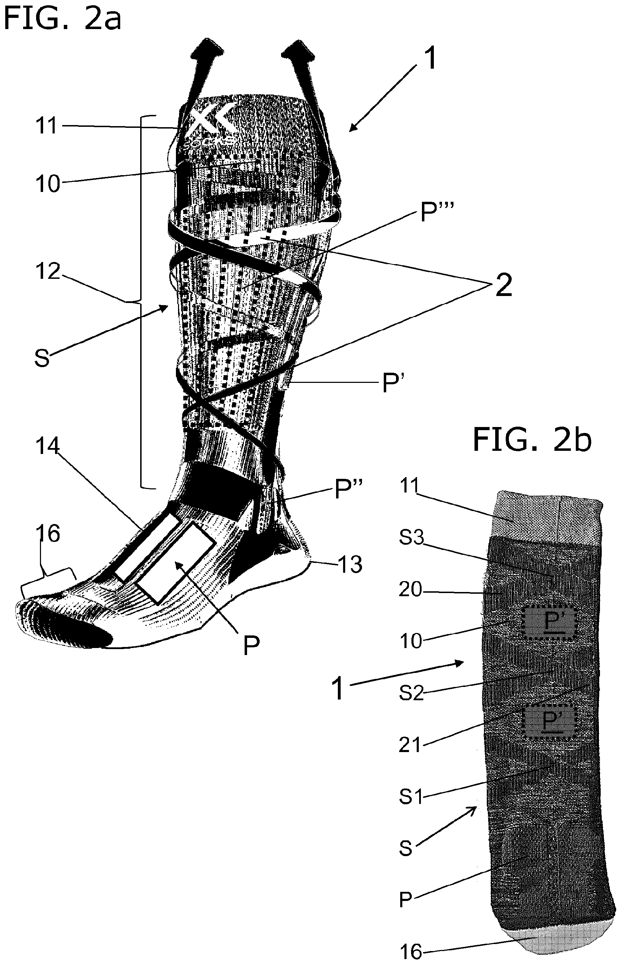

In Figur 2a ist ein Kompressionsstrumpf 1 gezeigt, welcher neben der bereits beschriebenen Ausgestaltung der umlaufenden Kompressionsmittel 2 zusätzliche Polster P, P', P", P‴ auf weist, wobei wahlweise mindestens ein zusätzliches Polster am Grundgestrick 10 befestigt oder angeformt bzw. in das Grundgestrick 10 integriert ist. Hier sind zweit Polster P im Bereich des Fussrückenbereiches 14 des Kompressionsstrumpfes 1 angebracht. Es sind aber auch Polster P' entlang des Wadenbereichs 120, Polster P" im Sprunggelenksbereich 121 und/oder Polster P‴ im Bereich des Schienbeins sinnvoll für den Einsatz im Sportbereich. Durch die Polster P, P', P", P‴ wird zusätzlich auch ein teilweise flächiger Druck erzeugt.

Bevorzugt wird entlang einer Vorderseite des Kompressionsstrumpfes 1, hier eines Schienbeinbereichs, das Polster P‴ vom Strumpfbund 11 bis zum Fussrückenbereich 14 ragend am Grundgestrick 10 angeordnet. Dabei kann das Polster P‴ die umlaufenden Kompressionsmittel 2 überdecken, sodass die kreuzenden Helices 20, 21 im Schienbeinbereich nicht erkennbar sind, da sie vom Polster P‴ überdeckt sind. Die Kompressionswirkung der Helices 20, 21 wird durch dieses Polster P‴ noch zusätzlich verstärkt.

In einer Ansicht gemäss Figur 2b auf einen Kompressionsstrumpf 1 im gefalteten Zustand sind hier entlang der kreuzenden Helices 20, 21 drei Schnittpunkte S1, S2, S3 auf der Textilvorderseite S zu erkennen. Die Helices 20, 21 sind oberhalb des Polsters P und des Fussrückenbereichs 14 in Richtung Strumpfbund 11 verlaufend angeordnet, wobei die Helices 20, 21 nicht in den Strumpfbund 11 eindringen. Der Strumpfbund 11 ist mit einem anderen Strickmuster und/oder einem andersfarbigen Garn als das Grundgestrick 10 ausgeführt.

Im Wadenbereich 120 sind mehrere Polster P' bevorzugt zwischen den kreuzenden Helices 20, 21 verteilt, also zwischen den Kreuzungspunkten der Helices 20, 21 angeordnet.

Die Gestaltung der Helices 20, 21 ist hier bandförmig ausgeführt, wobei die Dicke entlang der Windungen variiert. Möglich ist aber auch eine konstante Dicke im gesamten Windungsverlauf auszuführen. Die Helices 20, 21 sind dabei mit einer anderen Struktur, also bevorzugt mit einem vom Grundgestrick 10 abweichenden Strickmuster ausgeführt. Wahlweise kann für die Helices 20, 21 ein anderes Garn, insbesondere ein dickeres und/oder flexibleres Garn, als für das Grundgestrick 10 gewählt werden. Entlang der Helices 20, 21 können rippenartige Strukturen in Längsrichtung der Helices 20, 21 oder senkrecht dazu gewählt werden, wodurch die Kompressionsmittel 2 den Druck auf die oberste Hautschicht lokal stark erhöhen können. Da durch Verwendung von Gestricken, welche in der Regel einen hohen Grad an Elastizität haben, ein elastisches Grundgestrick und elastische Helices 20, 21 erzeugbar sind, ist die Verwendung von flexiblem Garn nicht unbedingt nötig, aber möglich. Der Umlauf der Helices 20, 21 kann bevorzugt auf Höhe des Fussgelenkes, also den Gelenkbereiche 121 umschliessend, des Unterschenkels des Trägers ausgeführt sein. Dann findet eine gesteigerte Stützung im Bereich des Fussgelenks statt.

Anstelle eines Kompressionsstrumpfes 1 wird als erfindungsgemässer Sportsocken 1 mit einem röhrenförmigen Schaft 12 auch als Bandage für den Bereich der Ellenbogen- oder Kniegelenke eingesetzt werden. Der Schaft 12 weist mindestens eine erste Seite, anstelle einer Strumpfsohle 15 auf und auf der gegenüberliegenden Seite einen Bund 11. Zwischen dieser ersten Seite und der Strumpfsohle 15 verläuft entsprechend der röhrenförmige Schaft 12. Den Schaft 12 umlaufend, sind die mindestens zwei Helices 20, 21, sich mindestens einmal kreuzend angeordnet, wobei die Helices 20, 21 mit dem Basistextil 10 verbunden sind, ein anderes Strickmuster oder anderes Garn als das Basistextil 10 aufweisend. Die Helices 20, 21 sind bevorzugt ausschliesslich entlang der Länge des Sportsocken 1 oder der Sportbandage zwischen dem Fussrückenbereich 14 und einem Kniebereich verlaufend angeordnet.

Die Helces 20, 21 weisen ein anderes Strickmuster und/oder anderes Garn auf, welche am Basistextil 10 befestigt, insbesondere gestrickt befestigt sind. Bevorzugt werden die Kompressionsmittel 20, 21 beim Strick- oder Wirkvorgang des Basistextils 10 mit eingestrickt oder - gewirkt, sodass der Schaft 12 eine geschlossene röhrenförmige Hülle bildet. Wenn das Sportextil 1 als Bandage ausgeführt ist, dann ist eine erste Seite, anstelle einer Strumpfsohle 15 dem Bund 11 gegenüberliegend vorgesehen. Zwischen der ersten Seite und dem Bund 11 verläuft dann der Schaft 12 und entlang des Schafts 12 die Kompressionsmittel 2. Die Polster P, P', P", P‴ werden ebenfalls in das Basistextil 10 eingestrickt und liegen vereinzelt oder zusammenhängend neben den Helices 20, 21 oder überdecken diese. Die Polster P, P', P", P‴ können aus dickerem Garn, einem speziellen Strickmuster und/oder einer Mischung daraus hergestellt sein. Die Stützwirkung der Helices 20, 21 wird entsprechend durch die Polster optimiert.

### Bezugszeichenliste

- 1: Sportsocken oder Sportbandage / Kompressionsstrumpf aus Basistextil
10 Basistextil/Grundgestrick/Grundgewirk (erstes Strickmuster)
11 Bund /Strumpfbund, elastisch gestrickt
12 Schaft / Strumpfschaft
120 Wadenbereich
121 Gelenkbereich / Sprunggelenksbereich
13 Fersenbereich
14 Fussrückenbereich
15 Strumpfsohle
16 Zehenbereich
S Textilvorderseite
R Textilrückseite

- 2: Kompressionsmittel (hier Helices, mindestens zwei Helices)
20 erste Helix
21 zweite Helix
A Startpunkt
S1, S2, S3 Schnittpunkte
E Endpunkt
P Polster

## Patentansprüche

1. Sportsocken (1) oder Sportbandage aus einem gewirkten oder gestrickten Basistextil (10), mit einem von einem Strumpfbund (11) bis zu einem Fussrückenbereich (14) verlaufenden röhrenförmigen Strumpfschaft (12), an welchem im Verlauf des Strumpfschafts (12) Kompressionsmittel (2) angeordnet sind, welche eine vom Basistextil (10) abweichende Struktur, Garndicke oder Garnmaterial aufweisen, wobei als Kompressionsmittel (2) mindestens zwei gegenläufige Helices (20, 21), welche vom Fussrückenbereich (14) in Richtung des Strumpfbundes (11) den Strumpfschaft (12) umlaufend ausgestaltet sind, welche sich mindestens einmal kreuzen und mit dem Basistextil (10) verbunden sind, indem das Strickmuster bzw. das Garn der Helices (20, 21) am Basistextil (10) befestigt ist,
**dadurch gekennzeichnet, dass**
auf einer Vorderseite des Sportsockens (1) bzw. der Sportbandage entlang eines Schienbeinbereichs mindestens ein zusätzliches Polster (P‴) in das Basistextil (10) eingestrickt ist, welches vom Strumpfbund (11) zu einem Fussrückenbereich (14) ragt und dabei die umlaufenden sich kreuzenden Helices (20, 21) mindestens teilweise überdeckt oder entlang seiner Ausdehnung auf der Vorderseite ersetzt.

2. Sportsocken (1) oder Sportbandage nach Anspruch 1, wobei auf einer Rückseite des Sportsockens (1) bzw. der Sportbandage eine Mehrzahl von zusätzlichen Polstern (P') zwischen den Helices (20, 21) entlang eines Wadenbereiches (120) am Basistextil (10) zwischen Kreuzungspunkten der Helices (20, 21) verteilt angeordnet ist.

3. Sportsocken (1) oder Sportbandage nach einem der vorhergehenden Ansprüche, wobei beide Helices (20, 21) mehr als 1.5 volle Umdrehungen, insbesondere mehr als zwei volle Umdrehungen im Verlauf um den Strumpfschaft (12) aufweisen.

4. Sportsocken (1) oder Sportbandage nach einem der vorhergehenden Ansprüche, wobei beide Helices (20, 21) entlang ihres Verlaufes unterschiedliche Ganghöhen, als Höhe bei einer vollen Umdrehung jeder Helix (20, 21), aufweisen.

5. Sportsocken (1) oder Sportbandage nach einem der vorhergehenden Ansprüche, wobei beide Helices (20, 21) mehr als drei Kreuzungspunkte (S1 bis S3) entlang ihrer Verläufe aufweisen.

6. Sportsocken (1) oder Sportbandage nach einem der vorhergehenden Ansprüche, wobei eine Ganghöhe, als Höhe bei einer vollen Umdrehung jeder Helix (20, 21) mindestens einem Viertel der Länge zwischen dem Rand des Strumpfbundes (11) und dem unteren Rand eines dem Strumpfbund (11) gegenüberliegenden Ende des Strumpfschafts (12) bzw. der Strumpfsohle (15) entspricht.

7. Sportsocken (1) oder Sportbandage nach einem der vorhergehenden Ansprüche, wobei beide Helices (20, 21) an einem gemeinsamen Startpunkt (S) beginnen.

8. Sportsocken (1) oder Sportbandage nach einem der vorhergehenden Ansprüche, wobei beide Helices (20, 21) in einem gemeinsamen Endpunkt (E) enden.

9. Sportsocken (1) oder Sportbandage aus einem gewirkten oder gestrickten Basistextil (10), mit einem von einem Strumpfbund (11) bis zu einem Fussrückenbereich (14) verlaufenden röhrenförmigen Strumpfschaft (12), an welchem im Verlauf des Strumpfschafts (12) Kompressionsmittel (2) angeordnet sind, welche eine vom Basistextil (10) abweichende Struktur, Garndicke oder Garnmaterial aufweisen, wobei als Kompressionsmittel (2) mindestens zwei gegenläufige Helices (20, 21), welche vom Fussrückenbereich (14) in Richtung des Strumpfbundes (11) den Strumpfschaft (12) umlaufend ausgestaltet sind, welche sich mindestens einmal kreuzen und mit dem Basistextil (10) verbunden sind, indem das Strickmuster bzw. das Garn der Helices (20, 21) am Basistextil (10) befestigt ist,
**dadurch gekennzeichnet, dass**
auf einer Rückseite des Sportsockens (1) bzw. der Sportbandage entlang eines Wadenbereiches (120) eine Mehrzahl von zusätzlichen Polstern (P') zwischen den Helices (20, 21) entlang des Wadenbereiches (120) am Basistextil (10) zwischen Kreuzungspunkten der Helices (20, 21) verteilt angeordnet ist.

## Claims

1. Sports socks (1) or sports bandage made of a knitted or crocheted base textile (10), with a tubular sock shank (12) extending from a sock band (11) to a foot back region (14), on which compression means (2) are arranged in the course of the sock shank (12), which compression means (2) have a structure differing from the base textile (10), thickness or yarn material differing from the base textile (10), wherein at least two counter-rotating helices (20, 21) are configured as compression means (2), which helices (20, 21) run around the hosiery shank (12) from the foot back region (14) in the direction of the hosiery waistband (11), cross each other at least once and are connected to the base textile (10) in that the knitting pattern or the yarn of the helices (20, 21) is attached to the base textile (10),
**characterised in that**
at least one additional pad (P‴) is knitted into the base textile (10) on a front side of the sports sock (1) or sports bandage along a shin area, which pad projects from the sock band (11) to a foot dorsal area (14) and thereby at least partially covers the circumferential intersecting helices (20, 21) or replaces them along its extension on the front side.

2. Sports sock (1) or sports bandage according to claim 1, wherein on a rear side of the sports sock (1) or sports bandage a plurality of additional pads (P') are arranged distributed between the helices (20, 21) along a calf region (120) on the base textile (10) between crossing points of the helices (20, 21).

3. Sports socks (1) or sports bandage according to one of the preceding claims, wherein both helices (20, 21) have more than 1.5 full revolutions, in particular more than two full revolutions, along their course around the shank (12) of the sock.

4. Sports sock (1) or sports bandage according to any one of the preceding claims, wherein both helices (20, 21) have different pitch heights along their course, as height at one full revolution of each helix (20, 21).

5. Sports sock (1) or sports bandage according to any one of the preceding claims, wherein both helices (20, 21) have more than three crossing points (S1 to S3) along their courses.

6. Sports socks (1) or sports bandage according to any one of the preceding claims, wherein a pitch, as a height at one full turn of each helix (20, 21), corresponds to at least one quarter of the length between the edge of the sock band (11) and the lower edge of an end of the sock shank (12) or sock sole (15) opposite to the sock band (11).

7. Sports sock (1) or sports bandage according to any one of the preceding claims, wherein both helices (20, 21) start at a common starting point (S).

8. Sports sock (1) or sports bandage according to any one of the preceding claims, wherein both helices (20, 21) end at a common end point (E).

9. Sports socks (1) or sports bandage made of a knitted or crocheted base textile (10), with a tubular sock shank (12) extending from a sock band (11) to a foot dorsal region (14), on which compression means (2) are arranged in the course of the sock shank (12), which compression means (2) have a structure differing from the base textile (10), thickness or yarn material differing from the base textile (10), wherein at least two counter-rotating helices (20, 21) are configured as compression means (2), which helices (20, 21) run around the hosiery shank (12) from the foot back region (14) in the direction of the hosiery waistband (11), cross each other at least once and are connected to the base textile (10) in that the knitting pattern or the yarn of the helices (20, 21) is attached to the base textile (10),
**characterised in that**
on a rear side of the sports sock (1) or sports bandage along a calf region (120), a plurality of additional pads (P') are arranged between the helices (20, 21) along the calf region (120) on the base textile (10) distributed between crossing points of the helices (20, 21).

## Revendications

1. Chaussette de sport (1) ou bandage de sport en un textile de base (10) tricoté ou crocheté, avec une tige de chaussette (12) tubulaire s'étendant d'une bande de chaussette (11) à une zone de dos de pied (14), sur laquelle sont disposés des moyens de compression (2) dans le sens de la tige de chaussette (12), lesquels moyens de compression (2) présentent une structure différente du textile de base (10), une épaisseur ou un matériau de fil différent du textile de base (10), dans lequel au moins deux hélices (20, 21) tournant en sens inverse sont configurées comme moyens de compression (2), lesquelles hélices (20, 21) s'étendent autour de la tige de chaussette (12) depuis la zone du dos du pied (14) en direction de la ceinture de la chaussette (11), se croisent au moins une fois et sont reliées au textile de base (10) par le fait que le motif de tricotage ou le fil des hélices (20, 21) est fixé au textile de base (10),
**caractérisé en ce que**
au moins un rembourrage supplémentaire (P‴) est tricoté dans le textile de base (10) sur une face avant de la chaussette de sport (1) ou du bandage de sport le long d'une zone du tibia, lequel rembourrage fait saillie depuis la bande de la chaussette (11) jusqu'à une zone dorsale du pied (14) et recouvre ainsi au moins partiellement les hélices (20, 21) se croisant sur la circonférence ou les remplace le long de son extension sur la face avant.

2. Chaussette de sport (1) ou bandage de sport selon la revendication 1, dans lequel, sur un côté arrière de la chaussette de sport (1) ou du bandage de sport, une pluralité de coussinets supplémentaires (P') sont disposés répartis entre les hélices (20, 21) le long d'une région de mollet (120) sur le textile de base (10) entre les points de croisement des hélices (20, 21).

3. Chaussettes de sport (1) ou bandage de sport selon l'une des revendications précédentes, dans lesquelles les deux hélices (20, 21) présentent plus de 1,5 tours complets, en particulier plus de deux tours complets dans leur parcours autour de la tige du bas (12).

4. Chaussettes de sport (1) ou bandage de sport selon l'une des revendications précédentes, dans lequel les deux hélices (20, 21) présentent des pas différents le long de leur parcours, en tant que hauteur pour un tour complet de chaque hélice (20, 21).

5. Chaussettes de sport (1) ou bandages de sport selon l'une quelconque des revendications précédentes, dans lesquels les deux hélices (20, 21) présentent plus de trois points d'intersection (S1 à S3) le long de leur trajet.

6. Chaussettes de sport (1) ou bandage de sport selon l'une quelconque des revendications précédentes, dans lesquelles une hauteur de pas, en tant que hauteur pour un tour complet de chaque hélice (20, 21), correspond au moins au quart de la longueur entre le bord de la ceinture du bas (11) et le bord inférieur d'une extrémité de la tige du bas (12) ou de la semelle du bas (15) opposée à la ceinture du bas (11).

7. Chaussettes de sport (1) ou bandages de sport selon l'une des revendications précédentes, dans lesquels les deux hélices (20, 21) commencent à un point de départ commun (S).

8. Chaussettes de sport (1) ou bandage de sport selon l'une des revendications précédentes, dans lequel les deux hélices (20, 21) se terminent en un point d'extrémité commun (E).

9. Chaussettes de sport (1) ou bandage de sport constitué d'un textile de base (10) tricoté, avec une tige de bas (12) tubulaire s'étendant d'une ceinture de bas (11) jusqu'à une zone de dos de pied (14), sur laquelle sont disposés, sur le parcours de la tige de bas (12), des moyens de compression (2) qui ont une structure différente du textile de base (10), épaisseur de fil ou de matériau de fil, au moins deux hélices (20, 21) tournant en sens inverse étant conçues comme moyens de compression (2), lesquelles font le tour de la tige de bas (12) depuis la zone du dos du pied (14) en direction de la ceinture de bas (11), se croisent au moins une fois et sont reliées à l'extile de base (10), en ce que le modèle de tricotage ou le matériau de fil est conçu de manière à ce que les hélices (20, 21) ne se croisent pas. le fil des hélices (20, 21) est fixé au textile de base (10),
**caractérisé en ce que**
sur une face arrière de la chaussette de sport (1) ou du bandage de sport, le long d'une zone du mollet (120), une pluralité de rembourrages supplémentaires (P') est disposée entre les hélices (20, 21) le long de la zone du mollet (120) sur l'extil de base (10), répartie entre des points de croisement des hélices (20, 21).
